(19) Europäisches Patentamt
European Patent Office
Office européen des brevets

(11) **EP 4 151 250 A1**

(12) **EUROPEAN PATENT APPLICATION**
published in accordance with Art. 153(4) EPC

(43) Date of publication:
**22.03.2023 Bulletin 2023/12**

(21) Application number: **21808827.6**

(22) Date of filing: **18.05.2021**

(51) International Patent Classification (IPC):
***A61M 1/16*** *(2006.01)*

(52) Cooperative Patent Classification (CPC):
**A61M 1/16**

(86) International application number:
**PCT/JP2021/018800**

(87) International publication number:
**WO 2021/235438 (25.11.2021 Gazette 2021/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.05.2020 JP 2020088045**

(71) Applicant: **Nipro Corporation
Osaka-shi, Osaka 531-8510 (JP)**

(72) Inventors:
• **NAKAMURA, Shota
Osaka-shi, Osaka 531-8510 (JP)**
• **KAMOSHITA, Yoichi
Osaka-shi, Osaka 531-8510 (JP)**
• **WASHIO, Yukio
Osaka-shi, Osaka 531-8510 (JP)**

(74) Representative: **Prüfer & Partner mbB
Patentanwälte · Rechtsanwälte
Sohnckestraße 12
81479 München (DE)**

(54) **BLOOD CIRCULATION MONITORING METHOD, DIALYSIS APPARATUS, AND PROGRAM**

(57) There is provided a technique for detecting leakage in extracorporeal blood circulation while suppressing increased cost in a dialysis device. A dialysis device (1) includes an artery-side blood circuit (110), a dialyzer (103), and a vein-side blood circuit (120). The dialysis device (1) detects an abnormality based on a difference between theoretical value and measured value of a blood concentration in the vein-side blood circuit (120). The theoretical value of the blood concentration in the vein-side blood circuit (120) is specified based on blood concentration and blood flow rate in the artery-side blood circuit (110) and a water removal rate in the dialyzer (103).

FIG.1

EP 4 151 250 A1

## Description

### TECHNICAL FIELD

The present disclosure relates to monitoring of a manner of extracorporeal blood circulation.

### BACKGROUND ART

Conventionally, there have been proposed various techniques for extracorporeal blood circulation in dialysis treatment or the like. For the extracorporeal blood circulation, Domestic Re-publication of PCT International Publication No. 2015-141621 (PTL 1) discloses a method for calibrating a blood concentration measurement value including an offset amount resulting from a difference between resin tubes, for example.

### CITATION LIST

### PATENT LITERATURE

PTL 1: Domestic Re-publication of PCT International Publication No. 2015-141621

### SUMMARY OF INVENTION

### TECHNICAL PROBLEM

In dialysis treatment, water removal is performed using a closed system; however, in the closed system, the water removal may be excessive or insufficient due to leakage at an electromagnetic valve or the like. It is considered that by providing a dialysis device with a mechanism for detecting leakage at each electromagnetic valve, the above-described excessive water removal and insufficient water removal can be detected. However, addition of such a mechanism may lead to increased cost in the dialysis device. Further, leakage occurring at a portion other than the electromagnetic valve, such as leakage at a water removal pump, is not detected by the mechanism.

The present disclosure has been conceived in view of such an actual circumstance and has an object to provide a technique for detecting leakage in extracorporeal blood circulation while suppressing increased cost in a dialysis device.

### SOLUTION TO PROBLEM

According to a certain aspect of the present disclosure, there is provided a computer-implemented method of monitoring blood circulation, the method including: obtaining a physical quantity in an artery-side blood circuit that allows blood to flow into a blood purifier; obtaining a physical quantity in a vein-side blood circuit that allows the blood to flow out of the blood purifier; obtaining, by using at least one of the physical quantity obtained in the artery-side blood circuit and the physical quantity obtained in the vein-side blood circuit, a theoretical value of the physical quantity in the artery-side blood circuit or the vein-side blood circuit to determine an abnormality of a mechanism that supplies a dialysis fluid to the blood purifier; determining occurrence of the abnormality based on the theoretical value, the physical quantity in the artery-side blood circuit or the vein-side blood circuit, and a given threshold value; and performing an operation abnormality process when it is determined that the abnormality has occurred.

The obtaining of the theoretical value may include calculating a theoretical value of an indicator of a blood concentration in the vein-side blood circuit based on an indicator of a blood concentration obtained in the artery-side blood circuit. The determining of the occurrence of the abnormality may include determining that the abnormality has occurred when a difference between the theoretical value and an indicator of a blood concentration obtained in the vein-side blood circuit is more than or equal to the given threshold value.

The obtaining of the theoretical value may include finding a theoretical value of a blood flow rate in the vein-side blood circuit based on a blood flow rate obtained in the artery-side blood circuit. The determining of the occurrence of the abnormality may include determining that the abnormality has occurred when a difference between the theoretical value and a blood flow rate obtained in the vein-side blood circuit is more than or equal to the given threshold value.

A flow rate of a blood pump provided to send the blood in the artery-side blood circuit may be used as the blood flow rate obtained in the artery-side blood circuit.

The theoretical value may be further based on a water removal rate in the blood purifier.

The obtaining of the theoretical value may include storing, into a storage device as the theoretical value, an indicator of a blood concentration obtained in the artery-side blood circuit or the vein-side blood circuit at a first timing at which a dialysis fluid pump that promotes discharging of the dialysis fluid from the blood purifier is not operated.

The determining of the occurrence of the abnormality may include determining that the abnormality has occurred when a difference between the theoretical value and the indicator obtained at a second timing at which the dialysis fluid pump is not operated is more than or equal to the given threshold value.

The determining of the abnormality may be performed before starting dialysis treatment using the blood purifier.

The obtaining of the theoretical value may include obtaining a first difference that is a difference between an indicator of a blood concentration obtained in the artery-side blood circuit at a first timing and an indicator of a blood concentration obtained in the vein-side blood circuit at the first timing. The determining of the occurrence of the abnormality may include obtaining a second difference that is a difference between an indicator of a blood concentration obtained in the artery-side blood circuit at a second timing and an indicator of a blood concentration obtained in the vein-side blood circuit at the second timing, and determining that the abnormality has occurred when a difference between the first difference and the second difference is more than or equal to the given threshold value.

A water removal rate in the blood purifier at the first timing may be equal to a water removal rate in the blood purifier at the second timing.

Each of the indicators of the blood concentrations may include hematocrit. According to another aspect of the present disclosure, there is provided a dialysis device including: a blood purifier; an artery-side blood circuit that allows blood to flow into the blood purifier; a vein-side blood circuit that allows the blood to flow out of the blood purifier; a blood pump provided to send the blood in the artery-side blood circuit; and a controller that controls an operation of the blood pump, wherein the controller performs the above-described method of monitoring the blood circulation.

According to still another aspect of the present disclosure, there is provided a program for causing, when executed by a computer, the computer to perform the above-described method of monitoring the blood circulation.

ADVANTAGEOUS EFFECTS OF INVENTION

According to the present disclosure, occurrence of an abnormality such as leakage in extracorporeal blood circulation is detected using a physical quantity in an artery-side blood circuit and/or a physical quantity in a vein-side blood circuit.

BRIEF DESCRIPTION OF DRAWINGS

Fig. 1 is a diagram showing an exemplary configuration of a dialysis device.
Fig. 2 is a diagram showing an exemplary formula representing a relation between Ht(A) and Ht(V).
Fig. 3 is a flowchart of a process performed by a dialysis device 1 to detect an abnormality in accordance with a first method.
Fig. 4 is a diagram for illustrating a second method for detecting an abnormality associated with leakage.
Fig. 5 is a flowchart of a process performed by dialysis device 1 to detect an abnormality in accordance with a second method.
Fig. 6 is a diagram for illustrating a third method for detecting an abnormality associated with leakage.
Fig. 7 is a flowchart of a process performed by dialysis device 1 to detect an abnormality in accordance with the third method.
Fig. 8 is a diagram for illustrating a fourth method for detecting an abnormality associated with leakage.
Fig. 9 is a flowchart of a process performed by dialysis device 1 to detect an abnormality in accordance with the fourth method.

DESCRIPTION OF EMBODIMENTS

Hereinafter, one embodiment of a dialysis device will be described with reference to figures. In the description below, the same parts and components are denoted by the same reference characters. Their names and functions are also the same. Therefore, these will not be described repeatedly.

[1. Configuration of Dialysis Device]

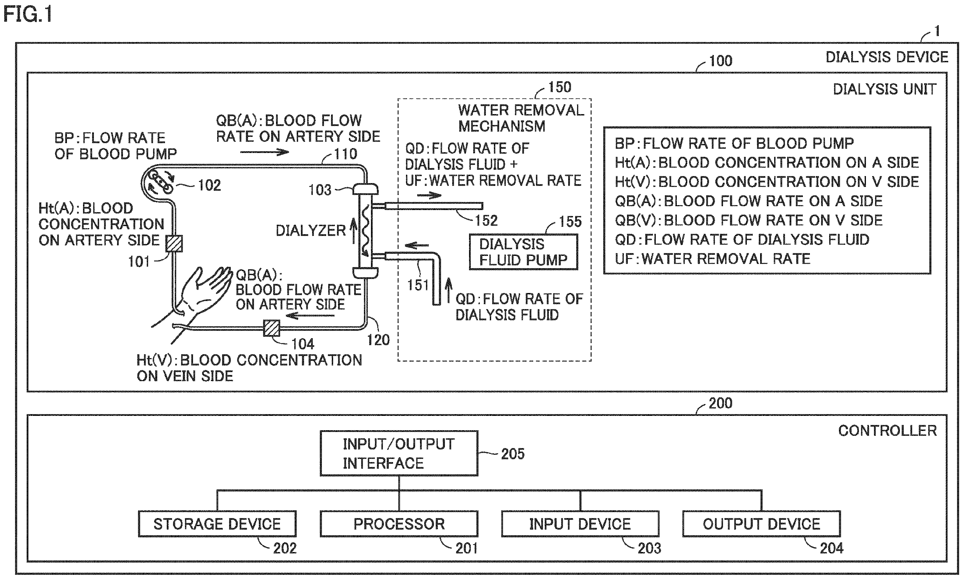

Fig. 1 is a diagram showing an exemplary configuration of a dialysis device. As shown in Fig. 1, a dialysis device 1 includes a dialysis unit 100 and a controller 200.

(Dialysis Unit 100)

Dialysis unit 100 includes: a dialyzer 103; an artery-side blood circuit 110 that connects a patient's artery to dialyzer 103; and a vein-side blood circuit 120 that connects the patient's vein to dialyzer 103. Dialyzer 103 is an exemplary blood purifier. Dialysis device 1 further includes: a blood pump 102 that sends blood to dialyzer 103 in artery-side blood circuit 110; a concentration measurement device 101 that measures a blood concentration in artery-side blood circuit 110; and a concentration measurement device 104 that measures a blood concentration in vein-side blood circuit 120.

Dialysis device 1 further includes a water removal mechanism 150, which is a mechanism that supplies a dialysis fluid to dialyzer 103. In water removal mechanism 150, the dialysis fluid is supplied to dialyzer 103 via an upstream-side dialysis fluid line 151, and the dialysis fluid is discharged from dialyzer 103 via a downstream-side dialysis fluid line 152. Water removal mechanism 150 includes a dialysis fluid pump 155 that promotes discharging of the dialysis fluid from dialyzer 103. As a method of supplying the dialysis fluid by water removal mechanism 150, various types of supply methods can be employed.

(Controller 200)

Controller 200 includes a processor 201, a storage device 202, an input device 203, an output device 204, and an input/output interface 205.
Processor 201 executes a program stored in storage device 202. Storage device 202 is constituted of a hard disk drive, a solid state drive, or the like. Storage device 202 may store various data to be used to execute a program.

Input device 203 is used by a user to input information to dialysis device 1, and is implemented by, for example, a keyboard, a mouse, a hardware button, and/or a touch sensor.

Output device 204 is used to output information from dialysis device 1, and is implemented by, for example, a display, an LED (Light Emitting Diode) lamp, and/or a speaker.

Input/output interface 205 is an interface for outputting data from each element in dialysis unit 100 to controller 200 and outputting data from controller 200 to each element in dialysis unit 100. In one example, controller 200 outputs a control instruction to blood pump 102 via input/output interface 205. In another example, controller 200 obtains respective measurement results from concentration measurement devices 101, 104 via input/output interface 205.

In the present embodiment, controller 200 controls dialysis unit 100 and can detect an abnormality associated with leakage in water removal mechanism 150. That is, controller 200 can detect abnormalities when leakages occur at various locations including an electromagnetic valve and dialysis fluid pump 155 of water removal mechanism 150.

[2. Water Removal in Dialysis Device]

Next, water removal from blood in dialysis device 1 will be described. In the description below, the names of physical quantities associated with dialysis device 1 are defined as follows.

"BP" represents a flow rate of blood pump 102.

"Ht(A)" represents a blood concentration on the artery side (A side). In the present embodiment, hematocrit is used as an exemplary indicator of the blood concentration. It should be noted that any other type of indicator may be employed as the indicator of the blood concentration.

"Ht(V)" represents a blood concentration on the vein side (V side).

"QB(A)" represents a blood flow rate on the artery side (A side).

"QB(V)" represents a blood flow rate on the vein side (V side).

"QD" represents a flow rate of a dialysis fluid sent from water removal mechanism 150 to dialyzer 103.

"UF" represents a water removal rate (water removal amount per unit) from the blood in dialyzer 103.

In dialysis device 1, water removal mechanism 150 sends the dialysis fluid at the flow rate "QD" to dialyzer 103, and discharges a solution (the dialysis fluid and a solution including the blood of the patient) at a flow rate "QD+UF" from dialyzer 103. In this way, an amount of the solution corresponding to the flow rate "UF" is removed from the blood of the patient.

More specifically, in dialysis device 1, blood pump 102 sends the blood at a flow rate Q per unit time from the artery of the patient's arm to dialyzer 103 via artery-side blood circuit 110. On the other hand, the dialysis fluid at flow rate QD per unit time is supplied to an inflow port of upstream-side dialysis fluid line 151 of dialyzer 103. Due to a concentration difference therebetween, an amount of water corresponding to flow rate LTF per unit time is removed from the blood flowing in a hollow fiber of dialyzer 103 to the dialysis fluid flowing around the hollow fiber of dialyzer 103. Then, the blood which has flow rate Q per unit time and from which the amount of water corresponding to flow rate UF has been removed is returned to the vein of the patient's arm via vein-side blood circuit 120. The removed amount of water corresponding to flow rate UF is discharged together with the dialysis fluid from dialyzer 103 via downstream-side dialysis fluid line 152.

[3. Overview of First Method]

A first method for detecting an abnormality associated with leakage will be described. In the first method, dialysis device 1 detects an abnormality based on a difference between theoretical value and measured value of a blood concentration in vein-side blood circuit 120. The theoretical value of the blood concentration in vein-side blood circuit 120 is specified based on the blood concentration and blood flow rate in artery-side blood circuit 110 and the water removal rate in dialyzer 103.

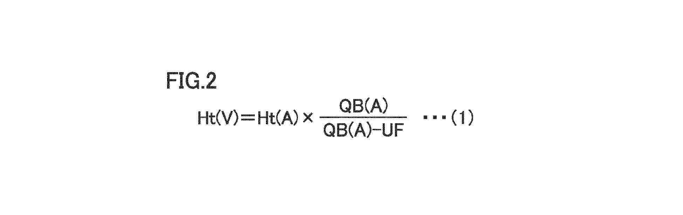

Fig. 2 is a diagram showing an exemplary formula representing a relation between Ht(A) and Ht(V). As shown as a formula (1) in Fig. 2, Ht(V) can be expressed as a value obtained by dividing the product of Ht(A) and QB(A) by "QB(A)-UF".

In dialysis device 1, each of UF and QB(A) is input from a user (for example, a medical worker such as a doctor) or registered in advance as a setting value. Controller 200 controls blood pump 102 in accordance with the setting value of QB(A) and controls dialysis fluid pump 155 in accordance with the setting value of UF.

Controller 200 obtains a measured value of Ht(A) and the setting values of LTF and QB(A) from concentration measurement device 101, thereby calculating a theoretical value of Ht(V) in accordance with the formula (1). Then, controller 200 obtains a measured value of Ht(V) from concentration measurement device 104, compares the measured value of Ht(V) with the theoretical value, and determines that an abnormality has occurred in water removal mechanism 150 when a difference of the theoretical value from the measured value of Ht(V) is more than or equal to a given threshold value.

The threshold value can be set for each situation to which the technique according to the present embodiment is applied. In one implementation, the threshold value is a value determined in advance and registered in dialysis device 1. In another implementation, the threshold value can be defined based on the theoretical value calculated on that occasion (for example, as a value of 10% of the theoretical value). That is, for example, when the difference between the measured value and the theoretical value is 10% or more of the theoretical value, controller 200 can determine that an abnormality has occurred in water removal mechanism 150.

[4. Flow of First Method]

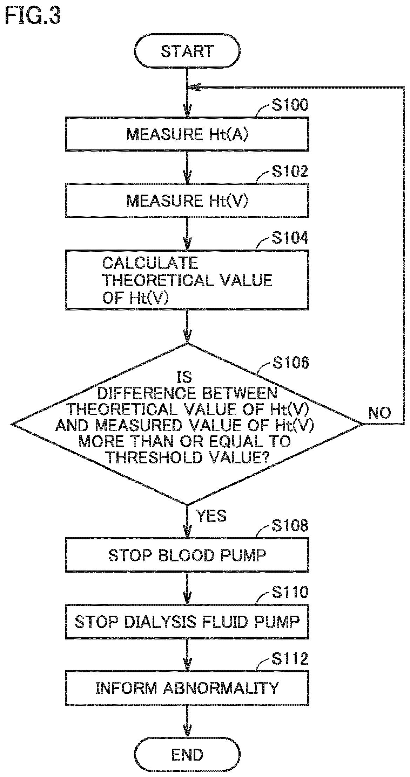

Fig. 3 is a flowchart of a process performed by dialysis device 1 to detect an abnormality in accordance with the first method. In one implementation, dialysis device 1 implements the process of Fig. 3 by causing processor 201 to execute a given program. Dialysis device 1 may include a dedicated circuit such as an ASIC (application specific integrated circuit) or FPGA (field-programmable gate array), and may implement the process of Fig. 3 as a function of the dedicated circuit.

Dialysis device 1 may start the process of Fig. 3 at certain time intervals (for example, every 10 seconds) during dialysis treatment, for example. During the dialysis treatment, dialysis device 1 may control an operation of blood pump 102 in accordance with a setting value and may control an operation of dialysis fluid pump 155 in accordance with a setting value. Each of the setting values for blood pump 102 and dialysis fluid pump 155 may be changed according to an elapsed time of the dialysis treatment.

Referring to Fig. 3, in a step S100, dialysis device 1 obtains a measured value of Ht(A) from concentration measurement device 101.

In a step S102, dialysis device 1 obtains a measured value of Ht(V) from concentration measurement device 104.

In a step S104, dialysis device 1 calculates a theoretical value of Ht(V) in accordance with the formula (1) using the measured value of Ht(A) obtained in step S100 and the setting values of QB(A) and UF.

In a step S106, dialysis device 1 determines whether or not a difference between the theoretical value of Ht(V) calculated in step S106 and the measured value of Ht(V) obtained in step S102 is more than or equal to a predetermined threshold value. When dialysis device 1 determines that the difference is less than the threshold value (NO in step S106), dialysis device 1 returns the control to step S100. Thus, for example, whenever a certain period of time has elapsed since the previous execution of step S100, dialysis device 1 executes the control of step S100 again. When dialysis device 1 determines that the difference is more than or equal to the threshold value (YES in step S106), dialysis device 1 proceeds the control to a step S108.

In step S108, dialysis device 1 stops blood pump 102.

In a step S110, dialysis device 1 stops dialysis fluid pump 155.

In a step S112, dialysis device 1 informs the abnormality and ends the process of Fig. 3. An example of the informing is presentation of information on the display serving as an exemplary output device 204. Another example thereof is output of sound from the speaker serving as an exemplary output device 204. Still another example thereof is transmission of a notification to an external device (for example, a mobile terminal carried by a medical worker).

In the first method described above, each of the stop of blood pump 102 (step S108), the stop of dialysis fluid pump 155 (step S110), and the informing of the abnormality (step S112) is an exemplary operation abnormality process.

[5. Overview of Second Method]

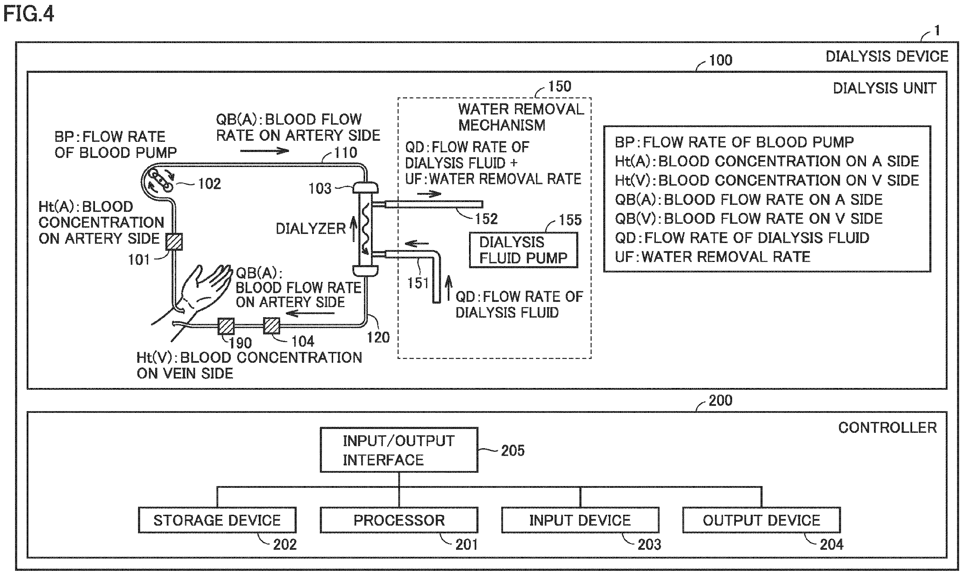

Fig. 4 is a diagram for illustrating a second method for detecting an abnormality associated with leakage. As shown in Fig. 4, dialysis device 1 may further include a flow meter 190. Flow meter 190 detects a flow rate (flow rate per unit time) of the blood in vein-side blood circuit 120.

Flow meter 190 is implemented by, for example, an ultrasonic flow meter, but may be a device that measures the flow rate of the blood in a different manner. Controller 200 obtains a measured value of QB(V) from flow meter 190.

In dialysis device 1, a relation between the blood flow rate in artery-side blood circuit 110 and the blood flow rate in vein-side blood circuit 120 can be expressed by the following formula (2):

$$QB(V)=QB(A)-UF \ldots (2)$$

In the second method, dialysis device 1 obtains respective setting values of blood pump 102 and dialysis fluid pump 155 as respective values of QB(A) and UF, thereby calculating a theoretical value of QB(V) in accordance with the formula (2). When a difference between the measured value of QB(V) and the theoretical value of QB(V) is more than or equal to a given threshold value, dialysis device 1 determines that an abnormality has occurred in water removal mechanism 150.

In one implementation, the threshold value is a value determined in advance and registered in dialysis device 1. In another implementation, the threshold value can be defined based on the theoretical value calculated on that occasion (for example, as a value of 10% of the theoretical value). That is, for example, when the difference between the measured value and the theoretical value is 10% or more of the theoretical value, controller 200 can determine that an abnormality has occurred in water removal mechanism 150.

[6. Flow of Second Method]

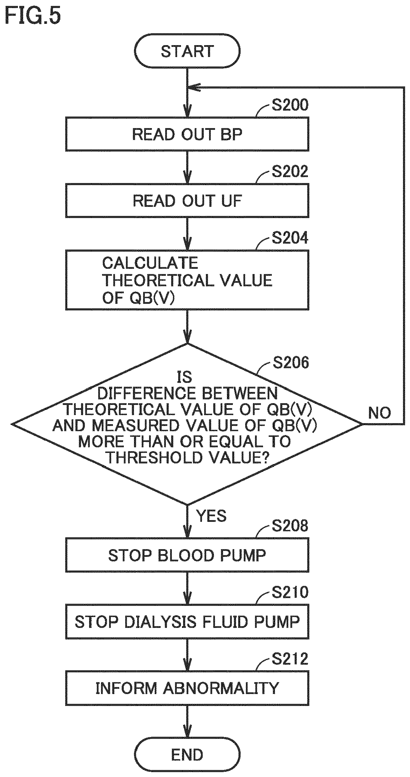

Fig. 5 is a flowchart of a process performed by dialysis device 1 to detect an abnormality in accordance with the second method. In one implementation, dialysis device 1 implements the process of Fig. 5 by causing processor 201 to execute a given program. Dialysis device 1 may include a dedicated circuit such as an ASIC or FPGA, and may implement the process of Fig. 5 as a function of the dedicated circuit.

Dialysis device 1 may start the process of Fig. 5 at certain time intervals (for example, every 10 seconds) during dialysis treatment, for example. During the dialysis treatment, dialysis device 1 may control an operation of blood pump 102 in accordance with a setting value and may control an operation of dialysis fluid pump 155 in accordance with a setting value. Each of the setting values for blood pump 102 and dialysis fluid pump 155 may be changed according to an elapsed time of the dialysis treatment.

Referring to Fig. 5, in a step S200, dialysis device 1 reads out a setting value of BP.

In a step S202, dialysis device 1 reads out a setting value of UF.

In a step S204, dialysis device 1 calculates a theoretical value of QB(V) in accordance with the formula (2) using BP and UF read out in step S200 and step S202. On this occasion, dialysis device 1 uses BP as a measured value of QB(A).

In a step S206, dialysis device 1 obtains a measured value of QB(V) from flow meter 190, and compares the measured value of QB(V) with the theoretical value of QB(V) calculated in step S204. Dialysis device 1 then determines whether or not a difference between the measured value and the theoretical value is more than or equal to a given threshold value.

When dialysis device 1 determines that the difference is less than the threshold value (NO in step S206), dialysis device 1 returns the control to step S200. Thus, for example, whenever a certain period of time has elapsed since the previous execution of step S200, dialysis device 1 executes the control of step S200 again. When dialysis device 1 determines that the difference is more than or equal to the threshold value (YES in step S206), dialysis device 1 proceeds the control to a step S208.

In step S208, dialysis device 1 stops blood pump 102.

In a step S210, dialysis device 1 stops dialysis fluid pump 155.

In a step S212, dialysis device 1 informs the abnormality and ends the process of Fig. 5. An example of the informing is presentation of information on the display serving as an exemplary output device 204. Another example thereof is output of sound from the speaker serving as an exemplary output device 204. Still another example thereof is transmission of a notification to an external device (for example, a mobile terminal carried by a medical worker).

In the second method described above, each of the stop of blood pump 102 (step S208), the stop of dialysis fluid pump 155 (step S210), and the informing of the abnormality (step S212) is an exemplary operation abnormality process.

[7. Overview of Third Method]

Next, a third method will be described. In the third method, dialysis device 1 detects an abnormality based on a change in a difference between measured value and theoretical value of blood concentration.

For example, in dialysis device 1, artery-side blood circuit 110 is connected to the artery of the patient, vein-side blood circuit 120 is connected to the vein of the patient, and water removal mechanism 150 and dialyzer 103 are connected to each other. In this state, blood pump 102 and dialysis fluid pump 155 have not been driven yet. That is, this state is an exemplary state before starting the dialysis treatment, i.e., when blood pump 102 and dialysis fluid pump 155 are started to be driven, the dialysis treatment is started.

In this state, it is considered that there is substantially no flow of blood and an amount of body fluid of the patient is not changed in the blood in each of artery-side blood circuit 110 and vein-side blood circuit 120. Therefore, when the blood concentration in artery-side blood circuit 110 or vein-side blood circuit 120 is changed in this state, an abnormality such as leakage is highly likely to have occurred in water removal mechanism 150.

In the third method, the blood concentration in artery-side blood circuit 110 is measured at each of a first timing and a second timing while keeping dialysis device 1 in the above-described state (state in which at least dialysis fluid pump 155 is not driven). When a difference between the blood concentration measured at the first timing and the blood concentration measured at the second timing is more than or equal to a given threshold value, dialysis device 1 determines that an abnormality such as leakage has occurred in water removal mechanism 150. On this occasion, the blood concentration measured at the first timing is used as an ideal value for the blood concentration measured at the second timing.

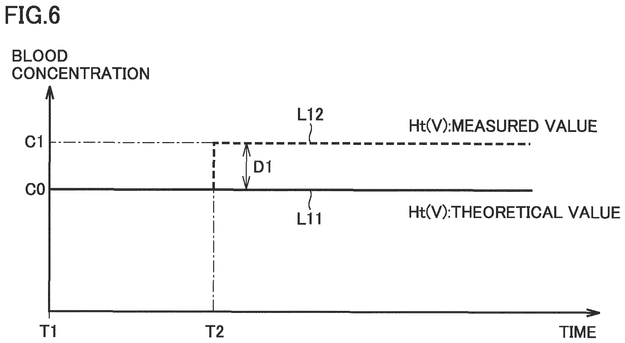

Fig. 6 is a diagram for illustrating the third method for detecting an abnormality associated with leakage. Fig. 6 shows an exemplary change in blood concentration with passage of time in the above-described state (state in which at least dialysis fluid pump 155 is not driven). A line L11 represents an ideal value of the blood concentration, and a line L12 represents an exemplary measured value of the blood concentration when an abnormality has occurred. When a blood concentration C1 measured at a time T2 (second timing) is changed by a threshold value or more with respect to a blood concentration C0 measured at a time T0 (first timing), dialysis device 1 determines that an abnormality has occurred.

In one implementation, the threshold value is a value determined in advance and registered in dialysis device 1. In another implementation, the threshold value may be defined based on the theoretical value calculated on that occasion (for example, as a value of 10% of the theoretical value). That is, for example, when a difference between the measured value and the theoretical value is 10% or more of the theoretical value, controller 200 can determine that an abnormality has occurred in water removal mechanism 150.

[8. Flow of Third Method]

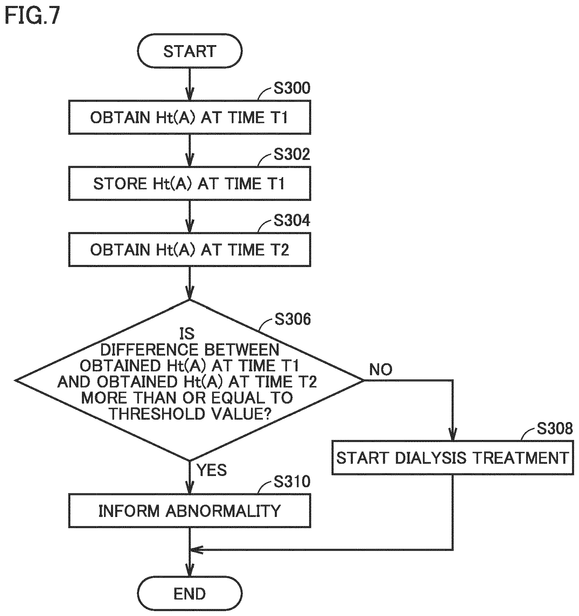

Fig. 7 is a flowchart of a process performed by dialysis device 1 to detect an abnormality in accordance with the third method. In one implementation, dialysis device 1 implements the process of Fig. 7 by causing processor 201 to execute a given program. Dialysis device 1 may include a dedicated circuit such as an ASIC or FPGA, and may implement the process of Fig. 7 as a function of the dedicated circuit.

Referring to Fig. 7, in a step S300, dialysis device 1 obtains a value (Ht(A)) measured by concentration measurement device 101 at time T1.

In a step S302, dialysis device 1 stores, into storage device 202, Ht(A) obtained in step S300.

In a step S304, dialysis device 1 obtains a value (Ht(A)) measured by concentration measurement device 101 at time T2.

In the third method, each of time T1 and time T2 can be set for each situation to which the third method is applied. In one implementation, time T1 and time T2 are in a period of time before starting dialysis treatment, and time T2 can be set as a timing at which a significant difference between the measured values at times T1, T2 is obtained when leakage has occurred in water removal mechanism 150.

In a step S306, dialysis device 1 determines whether or not a difference between Ht(A) at time T1 as stored in step S302 and Ht(A) obtained in step S304 is more than or equal to a given threshold value. When dialysis device 1 determines that the difference is less than the given threshold value (NO in step S306), dialysis device 1 starts the dialysis treatment in a step S308 and ends the process of Fig. 7. Thus, in dialysis device 1, an operation for the dialysis treatment, which includes driving of dialysis fluid pump 155, is started. On the other hand, when dialysis device 1 determines that the difference is more than or equal to the given threshold value (YES in step S306), dialysis device 1 proceeds the control to a step S310.

In step S310, dialysis device 1 informs the abnormality through presentation, sound, and/or notification to an external device, and ends the process of Fig. 7. The informing of the abnormality in step S310 is an exemplary operation abnormality process.

As the process of Fig. 7, dialysis device 1 detects an abnormality with the operation of dialysis fluid pump 155 being stopped, and informs the abnormality when the abnormality is detected (when the difference in step S306 is more than or equal to the given threshold value). In response to the informing of the abnormality, the user (for example, medical worker) checks water removal mechanism 150 including dialysis fluid pump 155, and handles matters such as repair and replacement of the electromagnetic valve as required.

In the above description, the blood concentration in artery-side blood circuit 110 is used to detect an abnormality; however, the blood concentration in vein-side blood circuit 120 may be used. That is, dialysis device 1 may obtain a measured value from concentration measurement device 104 at each of times T1 and T2. When a difference between these measured values is less than a predetermined threshold value, dialysis device 1 may start the dialysis treatment assuming that there is no abnormality, whereas when the difference is more than or equal to the threshold value, dialysis device 1 may inform the abnormality.

In the above description, dialysis device 1 performs the process of Fig. 7 before starting the dialysis treatment, and starts the dialysis treatment when no abnormality is detected. It should be noted that also during the dialysis treatment, dialysis device 1 may stop the operation of dialysis fluid pump 155 and may perform the process of Fig. 7 to thereby detect an abnormality. In this case, when an abnormality is detected, dialysis device 1 may perform a process of suspending the dialysis treatment (for example, stopping blood pump 102) before step S310.

[9. Overview of Fourth Method]

Next, a fourth method will be described. In the fourth method, dialysis device 1 detects an abnormality based on a change in difference between a blood concentration on the artery side and a blood concentration on the vein side.

More specifically, theoretically, when water removal rate UF is unchanged, the blood concentration on the artery side and the blood concentration on the vein side are unchanged in dialysis device 1. However, when leakage has occurred in water removal mechanism 150, an extra amount of water corresponding to an amount of leakage from the blood in dialyzer 103 is removed to increase the blood concentration on the downstream side with respect to dialyzer 103, i.e., on the vein side more than expected, with the result that the difference between the blood concentration on the artery side and the blood concentration on the vein side becomes larger than the theoretical one. Dialysis device 1 informs an abnormality when an amount of change in the difference between the blood concentration on the artery side and the blood concentration on the vein side is more than or equal to a given threshold value.

In one implementation, the threshold value is a value determined in advance and registered in dialysis device 1. In another implementation, the threshold value may be defined based on the difference on a given occasion (for example, as a value of 10% of the difference just before the start of the dialysis treatment or upon the start of the dialysis treatment). That is, for example, when the difference between the blood concentration on the artery side and the blood concentration on the vein side is changed by 10% or more with respect to the difference therebetween upon the start of the dialysis treatment, controller 200 can determine that an abnormality has occurred in water removal mechanism 150.

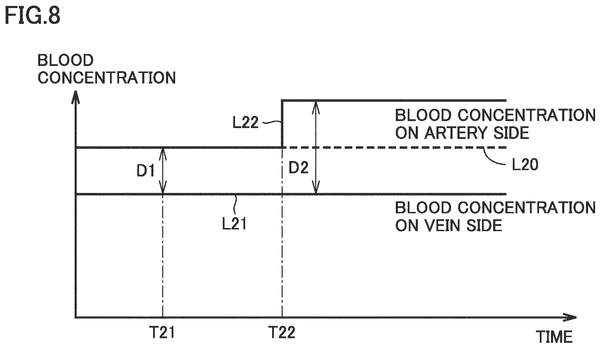

Fig. 8 is a diagram for illustrating a fourth method for detecting an abnormality associated with leakage. Fig. 8 shows a change in each of blood concentrations on the artery side and the vein side with passage of time. In Fig. 8, at a time T21, a difference between the blood concentration on the artery side and the blood concentration on the vein side is D1, whereas at a time T22, the difference between the blood concentration on the artery side and the blood concentration on the vein side is D2 that is larger than D1. When a difference between D1 and D2 is more than or equal to a given threshold value, dialysis device 1 can detect an abnormality in accordance with the change of the difference from D1 to D2.

[10. Flow of Fourth Method]

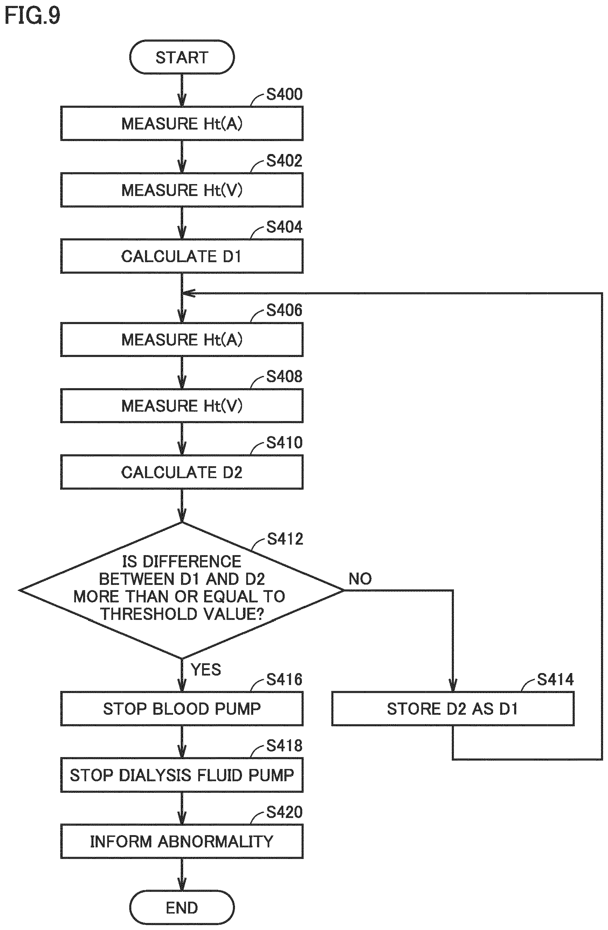

Fig. 9 is a flowchart of a process performed by dialysis device 1 to detect an abnormality in accordance with the fourth method. In one implementation, dialysis device 1 implements the process of Fig. 9 by causing processor 201 to execute a given program. Dialysis device 1 may include a dedicated circuit such as an ASIC or FPGA, and may implement the process of Fig. 9 as a function of the dedicated circuit.

Dialysis device 1 starts the process of Fig. 9 with starting of dialysis treatment, for example. During the dialysis treatment, dialysis device 1 may control an operation of blood pump 102 in accordance with a setting value and may control an operation of dialysis fluid pump 155 in accordance with a setting value. Each of the setting values for blood pump 102 and dialysis fluid pump 155 may be changed according to an elapsed time of the dialysis treatment.

Referring to Fig. 9, in a step S400, dialysis device 1 obtains a measured value (Ht(A)) from concentration measurement device 101.

In a step S402, dialysis device 1 obtains a measured value (Ht(V)) from concentration measurement device 104.

In a step S404, dialysis device 1 calculates a difference D1 between Ht(A) obtained in step S400 and Ht(V) obtained in step S402. Then, after passage of a predetermined time (for example, 10 seconds), dialysis device 1 proceeds the control to a step S406.

In step S406, dialysis device 1 obtains a measured value (Ht(A)) from concentration measurement device 101.

In a step S408, dialysis device 1 obtains a measured value (Ht(V)) from concentration measurement device 104.

In a step S410, dialysis device 1 calculates a difference D2 between Ht(A) obtained in step S406 and Ht(V) obtained in step S408.

In a step S412, dialysis device 1 determines whether a difference between D1 and D2 is more than or equal to a given threshold value. When dialysis device 1 determines that the difference is less than a given value (NO in step S412), dialysis device 1 proceeds the control to a step S414, whereas when dialysis device 1 determines that the difference is more than or equal to the given value (YES in step S412), dialysis device 1 proceeds the control to a step S416.

In step S414, dialysis device 1 stores, into storage device 202 as D1, the value calculated as difference D2 in step S410, and dialysis device 1 returns the control to step S406. Thus, after this, in step S412, D1 stored in storage device 202 in step S414 is compared with D2 calculated in step S410.

In step S416, dialysis device 1 stops blood pump 102.

In a step S418, dialysis device 1 stops dialysis fluid pump 155.

In a step S420, dialysis device 1 informs the abnormality and ends the process of Fig. 9. An example of the informing is presentation of information on the display serving as an exemplary output device 204. Another example thereof is output of sound from the speaker serving as an exemplary output device 204. Still another example thereof is transmission of a notification to an external device (for example, a mobile terminal carried by a medical worker).

In the fourth method described above, each of the stop of blood pump 102 (step S416), the stop of dialysis fluid pump 155 (step S418), and the informing of the abnormality (step S420) is an exemplary operation abnormality process.

In the fourth method, the difference between the blood concentration on the artery side (Ht(A)) and the blood concentration on the vein side (Ht(V)) is calculated for every predetermined time, and when the calculated difference is changed by a given threshold value or more with respect to the difference calculated previously, it is determined that an abnormality has occurred and an operation abnormality process is performed.

It should be noted that in the fourth method, step S414 may be omitted. In this case, in step S412, D2 calculated in step S410 is compared with D1 calculated in step S404. That is, D2 calculated for every predetermined time is compared with D1 calculated at the start of the process of Fig. 9 (for example, D1 upon the start of the dialysis treatment).

[11. Controller]

In the present embodiment, each of the processes described with reference to Figs. 3, 5, 7, and 9 is implemented by controller 200 of dialysis device 1 using an element such as concentration measurement device 101 in dialysis unit 100. In this sense, controller 200 may be implemented to be separated from dialysis device 1. That is, each of the processes described with reference to Figs. 3, 5, 7, and 9 may be implemented when a computer that can communicate with each element of dialysis device 1 and that are external to dialysis device 1 functions as controller 200.

The embodiments disclosed herein are illustrative and non-restrictive in any respect. The scope of the present invention is defined by the terms of the claims, rather than the embodiments described above, and is intended to include any modifications within the scope and meaning equivalent to the terms of the claims. The inventions described in the embodiments and the modifications are intended to be implemented solely or in combination wherever possible.

REFERENCE SIGNS LIST

1: dialysis device; 100: dialysis unit; 101, 104: concentration measurement device; 102: blood pump; 103: dialyzer; 110: artery-side blood circuit; 120: vein-side blood circuit; 150: water removal mechanism; 151: upstream-side dialysis fluid line; 152: downstream-side dialysis fluid line; 155: dialysis fluid pump; 190: flow meter; 200: controller.

**Claims**

1. A computer-implemented method of monitoring blood circulation, the method comprising:

   obtaining a physical quantity in an artery-side blood circuit that allows blood to flow into a blood purifier;
   obtaining a physical quantity in a vein-side blood circuit that allows the blood to flow out of the blood purifier;
   obtaining, by using at least one of the physical quantity obtained in the artery-side blood circuit and the physical quantity obtained in the vein-side blood circuit, a theoretical value of the physical quantity in the artery-side blood circuit or the vein-side blood circuit to determine an abnormality of a mechanism that supplies a dialysis fluid to the blood purifier;
   determining occurrence of the abnormality based on the theoretical value, the physical quantity in the artery-side blood circuit or the vein-side blood circuit, and a given threshold value; and
   performing an operation abnormality process when it is determined that the abnormality has occurred.

2. The method of monitoring the blood circulation according to claim 1, wherein

   the obtaining of the theoretical value includes calculating a theoretical value of an indicator of a blood concentration in the vein-side blood circuit based on an indicator of a blood concentration obtained in the artery-side blood circuit, and the determining of the occurrence of the abnormality includes determining that the abnormality has occurred when a difference between the theoretical value and an indicator of a blood concentration obtained in the vein-side blood circuit is more than or equal to the given threshold value.

3. The method of monitoring the blood circulation according to claim 1, wherein

   the obtaining of the theoretical value includes finding a theoretical value of a blood flow rate in the vein-side blood circuit based on a blood flow rate obtained in the artery-side blood circuit, and the determining of the occurrence of the abnormality includes determining that the abnormality has occurred when a difference between the theoretical value and a blood flow rate obtained in the vein-side blood circuit is more than or equal to the given threshold value.

4. The method of monitoring the blood circulation according to claim 3, wherein a flow rate of a blood pump provided to send the blood in the artery-side blood circuit is used as the blood flow rate obtained in the artery-side blood circuit.

5. The method of monitoring the blood circulation according to any one of claims 2 to 4, wherein the theoretical value is further based on a water removal rate in the blood purifier.

6. The method of monitoring the blood circulation according to claim 1, wherein

   the obtaining of the theoretical value includes storing, into a storage device as the theoretical value, an indicator of a blood concentration obtained in the artery-side blood circuit or the vein-side blood circuit at a first timing at which a dialysis fluid pump that promotes discharging of the dialysis fluid from the blood purifier is not operated, and
   the determining of the occurrence of the abnormality includes determining that the abnormality has occurred when a difference between the theoretical value and the indicator obtained at a second timing at which the dialysis fluid pump is not operated is more than or equal to the given threshold value.

7. The method of monitoring the blood circulation according to claim 6, wherein the determining of the abnormality is performed before starting dialysis treatment using the blood purifier.

**8.** The method of monitoring the blood circulation according to claim 1, wherein

the obtaining of the theoretical value includes obtaining a first difference that is a difference between an indicator of a blood concentration obtained in the artery-side blood circuit at a first timing and an indicator of a blood concentration obtained in the vein-side blood circuit at the first timing, and

the determining of the occurrence of the abnormality includes obtaining a second difference that is a difference between an indicator of a blood concentration obtained in the artery-side blood circuit at a second timing and an indicator of a blood concentration obtained in the vein-side blood circuit at the second timing, and determining that the abnormality has occurred when a difference between the first difference and the second difference is more than or equal to the given threshold value.

**9.** The method of monitoring the blood circulation according to claim 8, wherein a water removal rate in the blood purifier at the first timing is equal to a water removal rate in the blood purifier at the second timing.

**10.** The method of monitoring the blood circulation according to any one of claims 2 and 6 to 9, wherein each of the indicators of the blood concentrations includes hematocrit.

**11.** A dialysis device comprising:

a blood purifier;
an artery-side blood circuit that allows blood to flow into the blood purifier;
a vein-side blood circuit that allows the blood to flow out of the blood purifier;
a blood pump provided to send the blood in the artery-side blood circuit; and
a controller that controls an operation of the blood pump, wherein
the controller performs the method of monitoring the blood circulation according to any one of claims 1 to 10.

**12.** A program for causing, when executed by a computer, the computer to perform the method of monitoring the blood circulation according to any one of claims 1 to 10.

FIG.1

FIG.1

DIALYSIS DEVICE ~1

DIALYSIS UNIT ~100

WATER REMOVAL MECHANISM ~150

BP:FLOW RATE OF BLOOD PUMP

QB(A):BLOOD FLOW RATE ON ARTERY SIDE

QD:FLOW RATE OF DIALYSIS FLUID + UF:WATER REMOVAL RATE

Ht(A):BLOOD CONCENTRATION ON ARTERY SIDE

DIALYZER

QB(A): BLOOD FLOW RATE ON ARTERY SIDE

DIALYSIS FLUID PUMP ~155

QD:FLOW RATE OF DIALYSIS FLUID

Ht(V):BLOOD CONCENTRATION ON VEIN SIDE

BP:FLOW RATE OF BLOOD PUMP
Ht(A):BLOOD CONCENTRATION ON A SIDE
Ht(V):BLOOD CONCENTRATION ON V SIDE
QB(A):BLOOD FLOW RATE ON A SIDE
QB(V):BLOOD FLOW RATE ON V SIDE
QD:FLOW RATE OF DIALYSIS FLUID
UF:WATER REMOVAL RATE

CONTROLLER ~200

INPUT/OUTPUT INTERFACE ~205

STORAGE DEVICE ~202

PROCESSOR ~201

INPUT DEVICE ~203

OUTPUT DEVICE ~204

FIG.2

$$Ht(V) = Ht(A) \times \frac{QB(A)}{QB(A) - UF} \quad \cdots (1)$$

FIG.3

```
                    ( START )
                        │
                        │           S100
                        ▼        ┌──
              ┌──────────────────────┐
              │    MEASURE Ht(A)      │
              └──────────────────────┘
                        │           S102
                        ▼        ┌──
              ┌──────────────────────┐
              │    MEASURE Ht(V)      │
              └──────────────────────┘
                        │           S104
                        ▼        ┌──
              ┌──────────────────────┐
              │    CALCULATE         │
              │    THEORETICAL VALUE │
              │    OF Ht(V)          │
              └──────────────────────┘
                        │
                        ▼           S106
                       ╱╲        ┌──
                     ╱     ╲
                   ╱    IS    ╲
                 ╱ DIFFERENCE   ╲
                ╱   BETWEEN       ╲
               ╱ THEORETICAL VALUE ╲   NO
              ╱   OF Ht(V)          ╲────────┐
              ╲ AND MEASURED VALUE  ╱        │
               ╲  OF Ht(V)         ╱         │
                ╲ MORE THAN OR    ╱          │
                 ╲ EQUAL TO      ╱           │
                   ╲ THRESHOLD  ╱            │
                     ╲ VALUE? ╱              │
                       ╲╱                    │
                        │ YES                │
                        ▼        S108        │
              ┌──────────────────────┐       │
              │   STOP BLOOD PUMP    │       │
              └──────────────────────┘       │
                        │        S110        │
                        ▼                    │
              ┌──────────────────────┐       │
              │ STOP DIALYSIS FLUID  │       │
              │        PUMP          │       │
              └──────────────────────┘       │
                        │        S112        │
                        ▼                    │
              ┌──────────────────────┐       │
              │  INFORM ABNORMALITY  │       │
              └──────────────────────┘       │
                        │                    │
                        ▼                    │
                    (  END  )                │
```

FIG.4

DIALYSIS DEVICE

100 DIALYSIS UNIT

150 WATER REMOVAL MECHANISM

QB(A):BLOOD FLOW RATE ON ARTERY SIDE

BP:FLOW RATE OF BLOOD PUMP

Ht(A):BLOOD CONCENTRATION ON ARTERY SIDE

QB(A): BLOOD FLOW RATE ON ARTERY SIDE

QD:FLOW RATE OF DIALYSIS FLUID + UF:WATER REMOVAL RATE

DIALYZER

DIALYSIS FLUID PUMP

QD:FLOW RATE OF DIALYSIS FLUID

Ht(V):BLOOD CONCENTRATION ON VEIN SIDE

BP:FLOW RATE OF BLOOD PUMP
Ht(A):BLOOD CONCENTRATION ON A SIDE
Ht(V):BLOOD CONCENTRATION ON V SIDE
QB(A):BLOOD FLOW RATE ON A SIDE
QB(V):BLOOD FLOW RATE ON V SIDE
QD:FLOW RATE OF DIALYSIS FLUID
UF:WATER REMOVAL RATE

200 CONTROLLER

INPUT/OUTPUT INTERFACE — 205

STORAGE DEVICE — 202 | PROCESSOR — 201 | INPUT DEVICE — 203 | OUTPUT DEVICE — 204

EP 4 151 250 A1

FIG.5

```
                    ┌─────────────┐
                    │    START    │
                    └──────┬──────┘
                           │            ┌───────────┐
                           ▼  ╭─S200    │           │
                    ┌─────────────┐     │           │
                    │  READ OUT BP│     │           │
                    └──────┬──────┘     │           │
                           ▼  ╭─S202    │           │
                    ┌─────────────┐     │           │
                    │  READ OUT UF│     │           │
                    └──────┬──────┘     │           │
                           ▼  ╭─S204    │           │
                    ┌─────────────┐     │           │
                    │ CALCULATE   │     │           │
                    │ THEORETICAL │     │           │
                    │ VALUE       │     │           │
                    │ OF QB(V)    │     │           │
                    └──────┬──────┘     │           │
```

S206

IS DIFFERENCE BETWEEN THEORETICAL VALUE OF QB(V) AND MEASURED VALUE OF QB(V) MORE THAN OR EQUAL TO THRESHOLD VALUE?

NO

YES   S208

STOP BLOOD PUMP

S210

STOP DIALYSIS FLUID PUMP

S212

INFORM ABNORMALITY

END

FIG.6

FIG.7

```
          ┌─────────────┐
          │    START    │
          └──────┬──────┘
                 │         ┌S300
       ┌─────────▼─────────────┐
       │  OBTAIN Ht(A) AT TIME T1 │
       └─────────┬─────────────┘
                 │         ┌S302
       ┌─────────▼─────────────┐
       │  STORE Ht(A) AT TIME T1  │
       └─────────┬─────────────┘
                 │         ┌S304
       ┌─────────▼─────────────┐
       │  OBTAIN Ht(A) AT TIME T2 │
       └─────────┬─────────────┘
                 │
                 ▼           ┌S306
```

IS DIFFERENCE BETWEEN OBTAINED Ht(A) AT TIME T1 AND OBTAINED Ht(A) AT TIME T2 MORE THAN OR EQUAL TO THRESHOLD VALUE?

NO

S308

START DIALYSIS TREATMENT

YES

S310

INFORM ABNORMALITY

```
          ┌─────────────┐
          │     END     │
          └─────────────┘
```

FIG.8

FIG.9

START

S400
MEASURE Ht(A)

S402
MEASURE Ht(V)

S404
CALCULATE D1

S406
MEASURE Ht(A)

S408
MEASURE Ht(V)

S410
CALCULATE D2

S412
IS DIFFERENCE BETWEEN D1 AND D2 MORE THAN OR EQUAL TO THRESHOLD VALUE?

NO

YES

S416
STOP BLOOD PUMP

S414
STORE D2 AS D1

S418
STOP DIALYSIS FLUID PUMP

S420
INFORM ABNORMALITY

END

**INTERNATIONAL SEARCH REPORT**

| International application No. |
| --- |
| PCT/JP2021/018800 |

### A. CLASSIFICATION OF SUBJECT MATTER
A61M 1/16(2006.01)i
FI: A61M1/16 111

According to International Patent Classification (IPC) or to both national classification and IPC

### B. FIELDS SEARCHED

Minimum documentation searched (classification system followed by classification symbols)
A61M1/16

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X<br>A | JP 9-149935 A (NISSHO CO., LTD.) 10 June 1997<br>(1997-06-10) paragraphs [0007]-[0012] | 1-2, 5, 10-12<br>3-4, 6-9 |
| X<br>A | JP 2004-174235 A (NIKKISO CO., LTD.) 24 June 2004<br>(2004-06-24) paragraphs [0032]-[0040] | 1, 5, 10-12<br>2-4, 6-9 |
| A | JP 2013-27455 A (NIKKISO CO., LTD.) 07 February<br>2013 (2013-02-07) entire text, all drawings | 1-12 |
| A | JP 2009-543583 A (FRESENIUS MEDICAL CARE<br>DEUTSCHLAND GMBH) 10 December 2009 (2009-12-10)<br>entire text, all drawings | 1-12 |
| A | JP 54-6397 A (CORDIS DOW CORP.) 18 January 1979<br>(1979-01-18) entire text, all drawings | 1-12 |

☒ Further documents are listed in the continuation of Box C.      ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 05 August 2021 (05.08.2021) | 24 August 2021 (24.08.2021) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**

| International application No. |
| --- |
| PCT/JP2021/018800 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2016-027860 A (FRESENIUS MEDICAL CARE DEUTSCHLAND GMBH) 25 February 2016 (2016-02-25) entire text, all drawings | 1-12 |

Form PCT/ISA/210 (continuation of second sheet) (January 2015)

**INTERNATIONAL SEARCH REPORT**

| International application No. |
| --- |
| PCT/JP2021/018800 |

---

**Box No. II  Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III  Observations where unity of invention is lacking (Continuation of item 3 of first sheet)**

This International Searching Authority found multiple inventions in this international application, as follows:
   See extra sheet

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.  Consequently, this international search report is restricted to the invention first mentioned in the claims; it is covered by claims Nos.:

**Remark on Protest**
   ☐ The additional search fees were accompanied by the applicant's protest and, where applicable, the payment of a protest fee.
   ☐ The additional search fees were accompanied by the applicant's protest but the applicable protest fee was not paid within the time limit specified in the invitation.
   ☒ No protest accompanied the payment of additional search fees.

Form PCT/ISA/210 (continuation of first sheet (2)) (January 2015)

INTERNATIONAL SEARCH REPORT
Information on patent family members

International application No.

PCT/JP2021/018800

| Patent Documents referred in the Report | Publication Date | Patent Family | Publication Date |
|---|---|---|---|
| JP 9-149935 A | 10 Jun. 1997 | (Family: none) | |
| JP 2004-174235 A | 24 Jun. 2004 | US 2004/0129616 A1 paragraphs [0040]-[0048] EP 1566190 A1 | |
| JP 2013-27455 A | 07 Feb. 2013 | (Family: none) | |
| JP 2009-543583 A | 10 Dec. 2009 | US 2009/0292236 A1 entire text, all drawings WO 2008/006559 A1 | |
| JP 54-6397 A | 18 Jan. 1979 | DE 2825134 A1 entire text, all drawings | |
| JP 2016-027860 A | 25 Feb. 2016 | US 2012/0330214 A1 entire text, all drawings WO 2011/079941 A1 | |

Form PCT/ISA/210 (patent family annex) (January 2015)

**INTERNATIONAL SEARCH REPORT**

International application No.

PCT/JP2021/018800

<Continuation of Box No. III>

Document 1: JP 9-149935 A (NISSHO CO., LTD.) 10 June 1997 (1997-06-10) paragraphs [0007]-[0012] (Family: none)

Document 2: JP 2004-174235 A (NIKKISO CO., LTD.) 24 June 2004 (2004-06-24) paragraphs [0032]-[0040] & US 2004/0129616 A1 paragraphs [0040]-[0048] & EP 1566190 A1

Claims are classified into four inventions below.

(Invention 1) Claims 1-2, claims 5 and 10-12 referring to claim 2, claims 11-12 referring to claim 5, claims 11-12 referring to claim 10, claims 11-12 referring to claim 1

Document 1 cited in the international search report discloses a blood circulation monitoring method executed by a computer ("water removal quantity monitoring device") (particularly, refer to paragraphs [0007]-[0012]), the method comprising: a step for acquiring a physical amount in an artery side blood circuit ("blood concentration measured in an artery side line") for causing a blood to flow into a blood purifier (particularly, refer to paragraph [0011]); a step for acquiring a physical amount in a vein side blood circuit (blood concentration in a vein side line 6) for causing a blood to flow out from the blood purifier (particularly, refer to paragraph [0011]); a step for acquiring a theoretical value of a physical amount in the artery side blood circuit or the vein side blood circuit ("theoretical value of a blood concentration") for determining an abnormality in a mechanism for supplying a dialysate to the blood purifier by using at least one of the physical amount acquired in the artery side blood circuit or the physical amount acquired in the vein side blood circuit (particularly, refer to paragraph [0011]); a step for determining occurrence of the abnormality on the basis of the theoretical value, the physical amount in the artery side blood circuit or the vein side blood circuit, and a predetermined threshold value ("predetermined value") (particularly, refer to paragraphs [0008]-[0009]); and a step for executing a process for an operation abnormality in a case where it is determined that the abnormality has occurred (particularly, refer to "warning buzzer" in paragraphs [0008]-[0009]), and the invention in claim 1 lacks novelty in light of document 1, and thus does not have a special technical feature. Matters newly delimited in claims 2, 5, and 11 are indicated in document 1 (particularly, refer to paragraphs [0007]-[0012]), the invention in claims 2, 5, and 11 lacks novelty in light of document 1, and thus does not have a special technical feature. Therefore, the invention in claims 1-2, the invention in claim 5 referring to claim 2, and the invention in claim 11 referring to claim 5 are classified as invention 1.

Form PCT/ISA/210 (extra sheet) (January 2015)

**INTERNATIONAL SEARCH REPORT**

International application No.

PCT/JP2021/018800

Since the invention in claim 1 is a well-known feature (for example, refer to paragraphs [0007]-[0012] in document 1, and paragraphs [0032]-[0040] in document 2 cited in the international search report), when the technical feature of the invention initially set forth in the claim is grasped in consideration of other inventions classified as the invention 1, said technical feature is a blood circulation monitoring method executed by a computer, the method comprising: a step for acquiring a physical amount in an artery side blood circuit for causing a blood to flow into a blood purifier; a step for acquiring a physical amount in a vein side blood circuit for causing a blood to flow out from the blood purifier; a step for acquiring a theoretical value of a physical amount in the artery side blood circuit or the vein side blood circuit for determining an abnormality in a mechanism for supplying a dialysate to the blood purifier by using at least one of the physical amount acquired in the artery side blood circuit or the physical amount acquired in the vein side blood circuit; a step for determining occurrence of the abnormality on the basis of the theoretical value, the physical amount in the artery side blood circuit or the vein side blood circuit, and a predetermined threshold value; and a step for executing a process for an operation abnormality in a case where it is determined that the abnormality has occurred, wherein the step for the acquiring the theoretical value includes calculating a theoretical value of an index of a blood concentration in the vein side blood circuit on the basis of the index of the blood concentration acquired in the artery side blood circuit, and the step for the determining the occurrence of the abnormality includes determining that the abnormality occurs in a case where a difference between the index of the blood concentration acquired in the vein side blood circuit and the theoretical value is equal to or greater than a predetermined theoretical value. The invention in claim 12 referring to claim 5 dependent on claim 2, the invention in claims 10-12 referring to claim 2, and the invention in claims 11-12 referring to claim 10 have the technical feature of the invention initially set forth in the claim, and are inventively related to the technical feature of the invention initially set forth in the claim, and are thus classified as invention 1.

The invention in claims 11-12 referring to claim 1 is substantially identical or equivalent to the claims classified as invention 1, and thus is classified as invention 1.

(Invention 2) The invention in claims 3-4, the invention in claims 5, 11-12 referring to any one of claims 3-4, and the invention in claims 11-12 referring to claim 5

It cannot be said that the invention in claims 3-4, the invention in claims 5 and 11-12 referring to any one of claims 3-4, and the invention in claims 11-12 referring to claim 5 have the special technical feature identical or corresponding to claim 11 referring to all of claims 1-2 and 5.

Form PCT/ISA/210 (extra sheet) (January 2015)

**INTERNATIONAL SEARCH REPORT**

| International application No. |
|---|
| PCT/JP2021/018800 |

In the technical features of the invention in claims 3-4, the invention in claims 5 and 11-12 referring to any one of claims 3-4, and the invention in claims 11-12 referring to claim 5, which are added to claim 1, the step for the acquiring the theoretical value includes deriving a theoretical value of a blood flow rate in the vein side blood circuit on the basis of the blood flow rate acquired in the artery side blood circuit, and the step for the determining the occurrence of the abnormality includes determining that the abnormality occurs in a case where a difference between the blood flow rate acquired in the vein side blood circuit and the theoretical value is equal to or greater than a predetermined threshold value, and thus the invention initially set forth in the claim has low technical relevance with the technical feature. Therefore, it is not considered that the invention in claims 3-4, the invention in claims 5 and 11-12 referring to any one of claims 3-4, and the invention in claims 11-12 referring to claim 5 is inventively related to the invention initially set forth in the claims. Moreover, the invention in claims 3-4, the invention in claims 5 and 11-12 referring to any one of claims 3-4, and the invention in claims 11-12 referring to claim 5 are not substantially identical or equivalent to any of the claims classified as invention 1. The invention in claims 3-4, the invention in claims 5 and 11-12 referring to any one of claims 3-4, and the invention in claims 11-12 referring to claim 5 is a blood circulation monitoring method executed by a computer, the method comprising: a step for acquiring a physical amount in an artery side blood circuit for causing a blood to flow into a blood purifier; a step for acquiring a physical amount in a vein side blood circuit for causing a blood to flow out from the blood purifier; a step for acquiring a theoretical value of a physical amount in the artery side blood circuit or the vein side blood circuit for determining an abnormality in a mechanism for supplying a dialysate to the blood purifier by using at least one of the physical amount acquired in the artery side blood circuit or the physical amount acquired in the vein side blood circuit; a step for determining occurrence of the abnormality on the basis of the theoretical value, the physical amount in the artery side blood circuit or the vein side blood circuit, and a predetermined threshold value; and a step for executing a process for an operation abnormality in a case where it is determined that the abnormality has occurred, wherein the step for the acquiring the theoretical value includes deriving a theoretical value of a blood flow rate in the vein side blood circuit on the basis of the blood flow rate acquired in the artery side blood circuit, and the step for the determining the occurrence of the abnormality includes determining that the abnormality occurs in a case where a difference between the blood flow rate acquired in the vein side blood circuit and the theoretical value is equal to or greater than a predetermined theoretical value, and are thus classified as invention 2.

(Invention 3) The invention in claims 6-7, the invention in claims 10-12 referring to any one of claims 6-7, and the invention in claims 11-12 referring to claim 10

It cannot be said that the invention in claims 6-7, the invention in claims 10-12 referring to any one of claims 6-7, and the invention in claims 11-12 referring to claim 10 have the special technical feature identical or corresponding to claim 11 referring to all of claims 1-2 and 5.

**INTERNATIONAL SEARCH REPORT**

International application No.

PCT/JP2021/018800

In the technical features of the invention in claims 6-7, the invention in claims 10-12 referring to any one of claims 6-7, and the invention in claims 11-12 referring to claim 10, which are added to claim 1, the step for the acquiring the theoretical value includes storing the index of the blood concentration in the artery side blood circuit or the vein side blood circuit as the theoretical value in a storing device, the index being acquired at a first timing at which a dialysate pump for promoting a discharge of the dialysate from the blood purifier is not operated, and the step for the determining the occurrence of the abnormality includes determining that the abnormality occurs in a case where a difference between the theoretical value and the index acquired at a second timing at which the dialysate pump is not operated is equal to or greater than a predetermined threshold value. Therefore, the technical features have low technical relevance with the technical feature of the invention initially set forth in the claim. Therefore, it is not considered that the invention in claims 6-7, the invention in claims 10-12 referring to any one of claims 6-7, and the invention in claims 11-12 referring to claim 10 are inventively related to the invention initially set forth in the claim. The invention in claims 6-7, the invention in claims 10-12 referring to any one of claims 6-7, and the invention in claims 11-12 referring to claim 10 are not substantially identical or equivalent to any of the claims classified as invention 1 or invention 2. The invention in claims 6-7, the invention in claims 10-12 referring to any one of claims 6-7, and the invention in claims 11-12 referring to claim 10 is a blood circulation monitoring method executed by a computer, the method comprising: a step for acquiring a physical amount in an artery side blood circuit for causing a blood to flow into a blood purifier; a step for acquiring a physical amount in a vein side blood circuit for causing a blood to flow out from the blood purifier; a step for acquiring a theoretical value of a physical amount in the artery side blood circuit or the vein side blood circuit for determining an abnormality in a mechanism for supplying a dialysate to the blood purifier by using at least one of the physical amount acquired in the artery side blood circuit or the physical amount acquired in the vein side blood circuit; a step for determining occurrence of the abnormality on the basis of the theoretical value, the physical amount in the artery side blood circuit or the vein side blood circuit, and a predetermined threshold value; and a step for executing a process for an operation abnormality in a case where it is determined that the abnormality has occurred, wherein the step for the acquiring the theoretical value includes storing the index of the blood concentration in the artery side blood circuit or the vein side blood circuit as the theoretical value in a storing device, the index being acquired at a first timing at which a dialysate pump for promoting a discharge of the dialysate from the blood purifier is not operated, and the step for the determining the occurrence of the abnormality includes determining that the abnormality occurs in a case where a difference between the theoretical value and the index acquired at a second timing at which the dialysate pump is not operated is equal to or greater than a predetermined threshold value, and are thus classified as invention 3.

(Invention 4) The invention in claims 8-9, the invention in claims 10-12 referring to any one of claims 8-9, and the invention in claims 11-12 referring to claim 10

It cannot be said that the invention in claims 8-9, the invention in claims 10-12 referring to any one of claims 8-9, and the invention in claims 11-12 referring to claim 10 have the special technical feature identical or corresponding to claim 11 referring to all of claims 1-2 and 5.

Form PCT/ISA/210 (extra sheet) (January 2015)

**INTERNATIONAL SEARCH REPORT**

International application No.

PCT/JP2021/018800

In the technical features of the invention in claims 10-12 referring to any one of claims 8-9, and the invention in claims 11-12 referring to claim 10, which are added to claim 1, the step for the acquiring the theoretical value includes acquiring a first difference which is the difference between the index of the blood concentration in the artery side blood circuit and the index of the blood concentration in the vein side blood circuit, the difference being acquired at the first timing, and the step for the determining the occurrence of the abnormality includes acquiring a second difference which is the difference between the index of the blood concentration in the artery side blood circuit and the index of the blood concentration in the vein side blood circuit, the difference being acquired at the second timing, and determining that the abnormality occurs in a case where a difference between the first difference and the second difference is equal to or greater than a predetermined threshold value. Therefore, the technical features have low technical relevance with the technical feature of the invention initially set forth in the claim. Therefore, it is not considered that the invention in claims 8-9, the invention in claims 10-12 referring to any one of claims 8-9, and the invention in claims 11-12 referring to claim 10 are inventively related to the invention initially set forth in the claim. The invention in claims 8-9, the invention in claims 10-12 referring to any one of claims 8-9, and the invention in claims 11-12 referring to claim 10 are not substantially identical or equivalent to any of the claims classified as invention 1, invention 2, or invention 3. The invention in claims 8-9, the invention in claims 10-12 referring to any one of claims 8-9, and the invention in claims 11-12 referring to claim 10 is a blood circulation monitoring method executed by a computer, the method comprising: a step for acquiring a physical amount in an artery side blood circuit for causing a blood to flow into a blood purifier; a step for acquiring a physical amount in a vein side blood circuit for causing a blood to flow out from the blood purifier; a step for acquiring a theoretical value of a physical amount in the artery side blood circuit or the vein side blood circuit for determining an abnormality in a mechanism for supplying a dialysate to the blood purifier by using at least one of the physical amount acquired in the artery side blood circuit or the physical amount acquired in the vein side blood circuit; a step for determining occurrence of the abnormality on the basis of the theoretical value, the physical amount in the artery side blood circuit or the vein side blood circuit, and a predetermined threshold value; and a step for executing a process for an operation abnormality in a case where it is determined that the abnormality has occurred, wherein the step for the acquiring the theoretical value includes acquiring a first difference which is the difference between the index of the blood concentration in the artery side blood circuit and the index of the blood concentration in the vein side blood circuit, the difference being acquired at the first timing, and the step for the determining the occurrence of the abnormality includes acquiring a second difference which is the difference between the index of the blood concentration in the artery side blood circuit and the index of the blood concentration in the vein side blood circuit, the difference being acquired at the second timing and determining that the abnormality has occurred in a case where a difference between the first difference and the second difference is equal to or greater than a predetermined threshold value, and thus are classified as invention 4.

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015141621 A